(19) Europäisches Patentamt
European Patent Office
Office européen des brevets

(11) EP 2 647 391 B1

(12) EUROPEAN PATENT SPECIFICATION

(45) Date of publication and mention of the grant of the patent:
**02.05.2018 Bulletin 2018/18**

(21) Application number: **11845744.9**

(22) Date of filing: **29.09.2011**

(51) Int Cl.:
***A61K 49/00*** (2006.01) ***A61K 49/18*** (2006.01)

(86) International application number:
**PCT/JP2011/072431**

(87) International publication number:
**WO 2012/073588 (07.06.2012 Gazette 2012/23)**

# (54) MRI CONTRAST AGENT CONTAINING COMPOSITE PARTICLES

MRT-KONTRASTMITTEL MIT VERBUNDPARTIKELN

AGENT DE CONTRASTE POUR IRM CONTENANT DES PARTICULES COMPOSITES

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **02.12.2010 JP 2010269386**

(43) Date of publication of application:
**09.10.2013 Bulletin 2013/41**

(73) Proprietor: **Kaneka Corporation**
**Osaka-shi, Osaka 530-8288 (JP)**

(72) Inventors:
- **TAGO, Yoshiyuki**
  **Takasago-shi,**
  **Hyogo 676-8688 (JP)**
- **YOSHIDA, Shinichi**
  **Takasago-shi,**
  **Hyogo 676-8688 (JP)**
- **OHNO, Kohji**
  **Uji-shi**
  **Kyoto 611-0011 (JP)**
- **TSUJII, Yoshinobu**
  **Uji-shi**
  **Kyoto 611-0011 (JP)**
- **TABATA, Yasuhiko**
  **Kyoto-shi,**
  **Kyoto 606-8507 (JP)**

(74) Representative: **Vossius & Partner**
**Patentanwälte Rechtsanwälte mbB**
**Siebertstrasse 3**
**81675 München (DE)**

(56) References cited:
**WO-A1-2005/108451 JP-A- 2006 328 309**

- **PETRI PAPAPHILIPPOU ET AL: "Superparamagnetic Hybrid Micelles, Based on Iron Oxide Nanoparticles and Well-Defined Diblock Copolymers Possessing [beta]-Ketoester Functionalities", BIOMACROMOLECULES, vol. 10, no. 9, 14 September 2009 (2009-09-14), pages 2662-2671, XP55275164, US ISSN: 1525-7797, DOI: 10.1021/bm9005936**
- **BABU KOTHANDAPANI ET AL: "Synthesis of Polymer Grafted Magnetite Nanoparticle with the Highest Grafting Density via Controlled Radical Polymerization.", NANOSCALE RESEARCH LETTERS 2009, vol. 4, no. 9, 2009, pages 1090-1102, XP002758071, ISSN: 1931-7573**
- **Lina Gu et al.: "Synthesis of PPEGMEA-g-PMAA densely grafted double hydrophiliccopolymer and its use as a template for the preparation of size-controlledsuperparamagnetic Fe3O4/polymer nano-composites", Journal of Materials Chemistry, vol. 18 4 August 2008 (2008-08-04), pages 4332-4340, XP002758072, Retrieved from the Internet: URL:http://pubs.rsc.org/en/content/article pdf/2008/jm/b805841e?page=search [retrieved on 2016-05-25]**
- **HU FEIXIONG ET AL: "Cellular response to magnetic nanoparticles "PEGylated" via surface-initiated atom transfer radical polymerization.", BIOMACROMOLECULES MAR 2006, vol. 7, no. 3, March 2006 (2006-03), pages 809-816, XP002758073, ISSN: 1525-7797**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 2 647 391 B1

## Description

### Technical Field

The present invention relates to an MRI contrast agent containing composite particles.

### Background Art

In the MRI method (the magnetic resonance imaging method) that is one of the in vivo diagnostic imaging methods, contrast agents are often used in order to image specific tissues more clearly. The contrast agents are pharmaceutical agents for use in examinations to enhance the image contrast between a normal tissue and a target tissue in diagnosis and are usually used by intravenous injection. For the MRI contrast agents, generally, magnetic materials that affect nuclear magnetic resonance are used and, for example, gadolinium preparations and iron preparations are commercially available.

As compared to the gadolinium preparations, the iron preparations have the advantages of being excellent in biological safety and producing excellent imaging effects at low doses.

Generally, however, conventional iron preparations are taken into normal liver tissue in just a matter of minutes after intravenous administration but are not taken into tumor regions in the liver at all. Therefore, as MRI contrast agents, they can be used only for negative imaging of liver tumors. On the other hand, although iron preparations with a high stability in blood and low liver accumulation also have been clinically developed (for example, Patent Document 1), they are shown to be phagocytized mainly by macrophages and to have a high accumulation in lymph nodes and therefore are no different in terms of a tendency to be taken into the reticuloendothelial system. Thus, the conventional gadolinium preparations and iron preparations have problems in pharmacokinetics when being administered to biological bodies.

As an example of the methods in which the pharmacokinetics after administration to a biological body has been improved, a method of coating the surfaces of inorganic core nanoparticles with polymers through, for example, silane groups has been proposed (for example, Patent Document 2).

### Prior Art Documents

### Patent Documents

Patent Document 1: WO 2006/068653
Patent Document 2: Japanese Unexamined Patent Application Publication No. 2009-519316

BIOMACROMOLECULES, vol. 10, no. 9, 14 September 2009, pages 2662-2671, XP55275164 discloses superparamagnetic iron oxide nanoparticles with polymer graft chains bound to the surfaces of the particles at a very high density for steric stabilisation.

### Disclosure of Invention

### Problem to be Solved by the Invention

However, such conventional preparations have low dispersion stability and tend to be phagocytized by macrophages, which have been problems. Therefore, the present inventors aim to provide an MRI contrast agent that has high dispersion stability and improved functions and that tends not to be phagocytized by macrophages.

### Means for Solving Problem

The present invention is an MRI contrast agent containing composite particles, wherein the composite particles are those with polymer graft chains bound to surfaces of microparticles at a very high density sufficient to allow steric repulsion to occur between the graft chains, the microparticles are inorganic microparticles that exhibit superparamagnetism, and the polymer graft chains have a number average molecular weight (Mn) of 30,000 to 320,000, a graft density of 0.15 to 0.45 chain/nm2, and a molecular weight distribution of 1 to 1.5.

Effects of the Invention

The composite particles according to the present invention to which high molecular weight graft chains were bound at a very high density by living radical polymerization have high dispersion stability and tend not to be phagocytized by reticuloendothelial cells and therefore tend not to be accumulated in tissues such as a liver and a spleen. Furthermore, the above-mentioned composite particles are accumulated in tumors due to the enhanced permeability and retention effect (EPR effect). Moreover, they have a high relaxation level and therefore can exhibit an imaging effect (contrast) even at a lower concentration. The development of such a high-performance MRI contrast agent enables high-precision diagnostic imaging and can contribute to the development of advanced medical treatments.

Brief Description of Drawings

[FIG. 1] FIG. 1 is a transmission electron microscope (TEM) photograph of composite particles (Table 1, run 6) of an MRI contrast agent.

[FIG. 2] FIG. 2 is an MRI phantom image for measuring the relaxation level of the composite particles (Table 1, run 6) of the MRI contrast agent according to the present invention.

[FIG. 3] FIG. 3 is a picture of 100-fold dilutions of composite particles (Table 1, runs 3 to 5 and 8) of the MRI contrast agent according to the present invention, which was taken after they were stored at room temperature for six months.

[FIG. 4] FIG. 4 is a graph showing phagocytosis by macrophages of the composite particles (Table 1, runs 1 to 13) of the MRI contrast agent according to the present invention.

[FIG. 5A] FIG. 5A is a histopathological picture of liver of a mouse, to which composite particles (Table 1, run 5) of the MRI contrast agent according to the present invention were administered.

[FIG. 5B] FIG. 5B is a histopathological picture of liver of a mouse, to which composite particles (Table 1, run 8) of the MRI contrast agent according to the present invention were administered.

[FIG. 6] FIG. 6 is a graph showing the percentages of composite particles remaining in the blood of mice, to which the composite particles (Table 1, runs 6 and 9) of the MRI contrast agent according to the present invention were administered.

[FIG. 7A] FIG. 7A is a graph showing the rates of accumulation in tissues of mice, to which the composite particles (Table 1, runs 6 and 9) of the MRI contrast agent according to the present invention were administered.

[FIG. 7B] FIG. 7B is a graph showing the rates of accumulation in tissues per weight of mice, to which the composite particles (Table 1, runs 6 and 9) of the MRI contrast agent according to the present invention were administered.

[FIG. 8A] FIG. 8A is a picture showing the position of a tumor in the left femoral region of a tumor-bearing mouse, to which the composite particles (Table 1, run 6) of the MRI contrast agent according to the present invention are administered.

[FIG. 8B] FIG. 8B is an MRI contrast image of a mouse tumor before administration of the composite particles (Table 1, run 6) of the MRI contrast agent according to the present invention.

[FIG. 8C] FIG. 8C is an MRI contrast image of the mouse tumor taken 24 hours after administration of the composite particles (Table 1, run 6) of the MRI contrast agent according to the present invention.

Description of the Invention

The present inventors found that composite particles with polymer graft chains of a predetermined molecular weight bound to the surfaces of microparticles at a very high density sufficient to allow steric repulsion to occur between the graft chains were excellent in dispersion stability under physiological conditions over a prolonged period. Based on this finding, the present inventors solved the above-mentioned problems. As described above, with the excellent dispersion stability, the MRI contrast agent of the present invention has the advantages of (a) easy handling, (b) providing a stable drug effect, and (c) improved safety. Generally, since the MRI contrast agent is prepared in the medical setting immediately before being administered to a patient, the MRI contrast agent of the present invention that is easy to handle can reduce the time between preparation and administration and therefore is advantageous particularly for medical staff Furthermore, since contrast agents with composite particles that tend to aggregate (i.e., with low dispersion stability) are recognized as foreign bodies in the blood and are then metabolized and excreted, the drug effect is reduced and thereby a sufficiently high imaging effect is difficult to obtain. Therefore, the MRI contrast agent of the present invention with the excellent dispersion stability allows the drug effect to be stable. Moreover, there is a possibility that the contrast agents with composite particles that tend to aggregate (i.e., with low dispersion stability) may form a thrombus in the blood and therefore they have lower safety, while the MRI contrast agent of the present invention has high dispersion stability and therefore such a possibility is low, resulting in improved safety.

In the present specification, the phrase "the composite particles with excellent dispersion stability" denotes the state where macroscopically apparent aggregates and sediments of the composite particles are not observed in a conventional solvent. Furthermore, the phrase "composite particles are excellent in dispersion stability over a prolonged period" denotes the state where macroscopically apparent aggregates and sediments are not observed when the composite particles are stored in a solvent under normal storage conditions for a few months. In this context, the normal storage conditions denote conditions including a temperature around 18 to 30°C and a relative humidity of approximately 60% or lower. Preferably, the conventional solvent is an aqueous solvent such as a normal saline solution that is generally used for pharmaceutical preparations.

Generally, when the composite particles have higher graft chain molecular weights, the density of the graft chains at the chain end decreases as compared to the vicinity where the graft chains are bound to the microparticles. Therefore, it is expected that they tend to be phagocytized by reticuloendothelial cells. Contrary to this expectation, however, the composite particles contained in the MRI contrast agent of the present invention exhibit characteristics of tending not to be phagocytized by reticuloendothelial cells. The term "reticuloendothelial system" used in the present invention is a generic term for cells that are involved in biological defense by phagocytizing foreign bodies and typical examples thereof include macrophages.

Such composite particles make it possible to provide a MRI contrast agent that is typified by being accumulated in a tumor tissue through a blood circulating system and furthermore, that is characterized by depending on the pathological conditions in the tumor tissue and exhibiting the accumulation degree distribution that varies in the tumor tissue. The exhibition of the accumulation in the tumor tissue can be confirmed by the fact that in an MRI image, as compared to the test animal before the MRI contrast agent of the present invention is administered, the contrast is enhanced in the part corresponding to the tumor tissue of the test animal after administration.

That is, as described above, the present invention is an MRI contrast agent containing composite particles, wherein the composite particles are those with polymer graft chains bound to surfaces of microparticles at a very high density sufficient to allow steric repulsion to occur between the graft chains, the microparticles are inorganic microparticles that exhibit superparamagnetism, and the polymer graft chains have a number average molecular weight (Mn) of 30,000 to 320,000, a graft density of 0.15 to 0.45 chain/ nm2, and a molecular weight distribution of 1 to 1.5.

In the present invention, the "MRI contrast agent" is a substance (agent) that is administered in an in vivo diagnostic imaging method using an MRI (Magnetic Resonance Imaging) system that is used in clinical practice. When the substance is present in the object to be subjected to MRI measurement, how the measuring object looks in the measurement image seems to have been changed significantly as compared to the case of absence of the substance. Thus, contrast can be given to the image.

In the present invention, the term "polymer graft chains" denotes polymer chains, each of which is formed with at least two monomers elongated from the surface of a microparticle by a polymerization reaction. Furthermore, the term "a very high density" denotes the density of graft chains in the case where the graft chains have thickened to such an extent that steric repulsion occurs between the graft chains. In this case, the graft chains each take a form of substantially fully elongated in the direction perpendicular to the surface. Furthermore, the term "bind/bound" denotes a bond that is formed by a common chemical reaction and examples thereof include a covalent bond and an ionic bond.

In the MRI contrast agent of the present invention, the polymer graft chains have a number average molecular weight (Mn) of at least 30,000, preferably 30,000 to 320,000. By controlling the number average molecular weight of the graft chains, differences are found in phagocytosis of reticuloendothelial cells and retention in blood of the MRI contrast agent of the present invention can be controlled. Specifically, when the graft chains have a high number average molecular weight, the retention in blood of the MRI contrast agent improves. Furthermore, when the graft chains have a high number average molecular weight, the MRI contrast agent tends not to be phagocytized by reticuloendothelial cells. The polymer graft chains have a number average degree of polymerization of 10 to 100,000. For example, when the monomer has a molecular weight of 500, the number average degree of polymerization is preferably 50 to 500, more preferably 50 to 350, and most preferably 70 to 350. The number average molecular weight can be measured by, for example, GPC. The number average degree of polymerization can be calculated by dividing the number average molecular weight by the monomer molecular weight.

In the MRI contrast agent of the present invention, the microparticles are inorganic microparticles that exhibit superparamagnetism. Examples of such inorganic microparticles include iron (Fe), cobalt (Co), an alloy (for example, FePt and FeCo), and iron oxide (for example, ferrite and magnetite). Ferrite is preferable since it has been used successfully in commercial iron preparations for MRI contrast agents. The "ferrite" is a generic term for ceramics containing iron oxide as a main component thereof and is widely used as a magnetic material. The inorganic microparticles are more preferably ferrite having a spinel type crystal structure and further preferably magnetite ($Fe_3O_4$). In this context, those referred to as SPIO (a superparamagnetic iron oxide preparation), USPIO (an ultra-small SPIO), and MION (a monocrystalline iron oxide nanoparticle preparation) are included in the iron preparations for an MRI contrast agent.

The term "superparamagnetism" is a phenomenon that occurs in magnetic inorganic nanoparticles and denotes that when an external magnetic field is applied thereto, they are magnetized as a whole. The superparamagnetic substance has properties of nanoparticles obtained by miniaturizing a ferromagnetic substance to have a size that allows only a single magnetic domain to be contained. Unlike the ferromagnetic substance that remains magnetized (magnetic storage) even when the magnetic field is removed, the superparamagnetic substance loses magnetization when the magnetic field is removed. Therefore, preferably, the inorganic microparticles that exhibit superparamagnetism have a particle size of 50 nm or smaller. This particle size can be measured by, for example, transmission electron microscopy. Whether the composite particles of the present invention exhibit superparamagnetism can be confirmed with, for example, a SQUID (Superconducting Quantum Interference Device) fluxmeter (Macromolecules, 42(4), p1219-1228, 2009).

The inorganic microparticles that exhibit superparamagnetism affect the relaxation times (both $T_1$ and $T_2$) of nuclei (generally water protons in clinical MRI) that emit MR signals used for forming images in the MRI measurement. The iron preparation containing the inorganic microparticles emphasizes the contrast in $T_2$-weighted image by the effect of shortening mainly $T_2$ (strictly speaking, shortening T2* by inducing nonuniformity in the local magnetic field).

A transverse relaxation level ($R_2$) that is expressed in the reciprocal of $T_2$ is used as a value indicating the $T_2$ shortening rate of the contrast agent. The $R_2$ of the composite particles basically depends on magnetization of the inorganic microparticles that exhibit superparamagnetism and the $R_2$ of existing SPIO or USPIO is generally 20 to 200 [Fe $mM^{-1} \cdot s^{-1}$] (Advanced Drug Delivery Reviews, Vol.58, p1471-1504, 2006). A higher $R_2$ indicates a higher $T_2$ shortening effect at a lower concentration. When the inorganic microparticles that exhibit superparamagnetism are magnetite ($Fe_3P_4$) and are used as a negative contrast agent, the composite particles have a transverse relaxation level ($R_2$) of preferably at least 100 [Fe $mM^{-1} \cdot s^{-1}$] (magnetic field strength: 1.5T) and more preferably at least 200 [Fe $mM^{-1} \cdot s^{-1}$] (magnetic field strength: 1.5T).

In the MRI contrast agent of the present invention, the polymer graft chains have a graft density of 0.15 to 0.45 chain/$nm^2$. Controlling this graft density allows the retention in blood of the composite particles in the MRI contrast agent of the present invention to be controlled. In the present invention, the term "graft density" or "density" denotes the number of graft chains bound to the surface per unit area ($nm^2$) of the surfaces of the microparticles. The graft density (s) is calculated as follows by determining the amount of the polymer graft chains (the amount of grafts, w) that are formed by being elongated from the surfaces of microparticles, by elemental analysis and using the value thus obtained, the surface area (S, $nm^2$) of core microparticles, the number average molecular weight Mn and Avogadro's number (Av) of the graft polymers.

$$\text{Calculation Method}: s = (w/Mn)Av/S$$

In the MRI contrast agent of the present invention, the composite particles have an average particle size of preferably 1000 nm or smaller, more preferably 10 to 500 nm, and further preferably 10 to 250 nm. In the MRI contrast agent of the present invention, the retention in blood thereof seldom depends on the average particle size. Therefore, when having graft chains with a predetermined molecular weight, the MRI contrast agent of the present invention is not eliminated easily from a biological body even when the particle size of the microparticles is increased and as a result, the average particle size of the composite particles is increased. Consequently, the MRI contrast agent of the present invention is excellent in sensitivity as compared to conventional agents and can produce an effect at a low dose. The average particle size denotes the hydrodynamic diameter of a whole composite particle including graft chains provided for a core microparticle. Furthermore, in the case of composite particles in which the particle size distribution of the average particle size is small, the MRI contrast agent has a uniform size and therefore is preferable for DDS. The average particle size can be measured by a dynamic light scattering-type particle size distribution measuring method.

In the MRI contrast agent of the present invention, the polymer graft chains can be obtained, for example, by living radical polymerization of monomers using polymerization initiating groups located on the surfaces of microparticles as base points. The type of the monomers is preferably at least one selected from the group consisting of an acrylic acid derivative, a methacrylic acid derivative, an acrylamide derivative, a methacrylamide derivative, and a styrene derivative. However, it is not limited to them and can be selected suitably in the range recognized by those skilled in the art. The polymer graft chains may be bound to other polymer graft chains. Specifically, in the living radical polymerization, block copolymers are grafted using cross-linking monomers as one type of monomers and then the cross-linking groups thus introduced may be reacted. Furthermore, the living radical polymerization may be, for example, random polymerization, block polymerization, or of a composition gradient type. The polymer graft chains each may have a specific ligand introduced into an end thereof or the vicinity of the end. An MRI contrast agent containing composite particles having such ligands is preferable since it can selectively deliver the ligands to a target organ. The specific ligand is not particularly limited as long as it is a specific ligand known in the present field.

In the MRI contrast agent of the present invention, the polymer graft chains have preferably a molecular weight distribution (Mw/Mn) of 1 to 1.5. This is because when the molecular weight distribution is in the range of 1 to 1.5, the MRI contrast agent has homogeneous graft surfaces.

Furthermore, in the composite particles of the MRI contrast agent of the present invention, polymer graft chains located in a region distant from microparticles may be polymers directed to a specific diseased tissue. Examples of the polymers directed to a specific diseased tissue include target molecules for molecular markers that are highly expressed at the time of lesion development. Such an MRI contrast agent can also be accumulated specifically in a lesion other than tumor. As a result, such a lesion can be detected as an MRI signal contrast.

Preferably, the MRI contrast agent of the present invention is used for tumor diagnosis of, for example, a target affected part (an organ, a tissue, a cell, a pathogen, etc.). The MRI contrast agent of the present invention is characterized by inhibiting adsorption of a protein working for removing it from the blood and as a result, having a high percentage thereof remaining in the blood. Therefore, the MRI contrast agent of the present invention highly accumulates in cancer tissue. The possible reason for this is an EPR effect (Enhanced Permeability and Retention Effect). It is known that a substance with a molecular size of tens of nanometers to 250 nm accumulates due to anatomical features of blood vessels of cancer tissue. The MRI contrast agent of the present invention has high retention in blood and as a result, has a high probability to pass through the cancer tissue thorough the bloodstream. Consequently, it is considered that the MRI contrast agent accumulates more in cancer.

Furthermore, the MRI contrast agent of the present invention has a low transfer rate to liver and exhibits a property of accumulating in tumor. Therefore, it is possible to detect tumors in various organs by the MRI method using the MRI contrast agent of the present invention.

In the MRI contrast agent of the present invention, the composite particles can be produced, for example, as follows. First, a compound to be used as a coupling agent containing a polymerization initiating group is fixed to the surfaces of microparticles by a chemisorption method. Then at least one type of monomers is supplied thereto to perform living radical graft polymerization. With the coupling agent containing a polymerization initiating group being fixed to the surfaces of the microparticles beforehand, graft polymerization can be progressed on the surfaces of the microparticles, with the graft density maintained constant. That is, the amount of grafts can be increased in proportion to Mn (the number average molecular weight) of the graft chains, and polymerization is allowed to progress in a living manner and thereby almost all graft chains located on the surfaces of the microparticles are allowed to grow substantially evenly. That is, in the composite particles of the present invention, steric hindrance between adjacent graft chains located on the surfaces of the microparticles is reduced. Furthermore, during the living radical-graft polymerization, a polymerization initiator that is not fixed to the surfaces of the microparticles may be allowed to coexist.

The compound to be used as a coupling agent containing a polymerization initiating group can be selected in view of, for example, an affinity with the microparticles. Specifically, when the microparticles are iron oxide particles, the coupling agent containing a polymerization initiating group thereof is preferably a silane compound represented by the following formula (I).

[Chemical Formula 1]

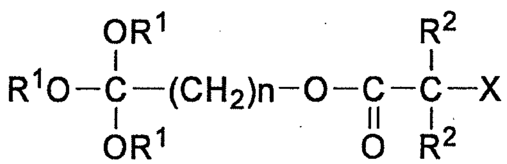
(I)

In the above formula, n denotes an integer and is preferably 3 to 10 and more preferably 4 to 8. $R^1$ is a $C_1$-$C_3$ alkyl group and is preferably a $C_1$ or $C_2$ alkyl group. $R^2$ is a $C_1$ or $C_2$ alkyl group. X is a halogen atom and is preferably a bromine or chlorine atom. Examples of the compound represented by Formula (I) include (2-bromo-2-methyl)propionyloxypropyltriethoxysilane (BPE) and (2-bromo-2-methyl)propionyloxyhexyltriethoxysilane (BHE).

When the microparticles are iron oxide, examples of the coupling agent that does not contain a polymerization initiating group thereof include an alkylsilane coupling agent.

It is possible to change the graft density on the surfaces of the microparticles by adjusting the ratio between the coupling agent containing a polymerization initiating group and the coupling agent that does not contain a polymerization initiating group. Specifically, in the case where the microparticles are iron oxide nanoparticles, the graft density is at least 0.1 chain/$nm^2$ when the ratio between the coupling agent containing a polymerization initiating group and the coupling agent that does not contain a polymerization initiating group is 1:0.

After completion of the polymerization, the intended composite particles can be isolated from the reaction solution by a method generally used in the present field, for example, by one of the methods such as extraction, distillation, washing, concentration, precipitation, filtration, drying, adsorption, deposition, and chromatography or a combination thereof

Preferably, the MRI contrast agent of the present invention is used for tumor diagnosis as described above. Examples of the tumor include non-neoplastic swellings and true tumors. The true tumors include a benign tumor and a malignant tumor. Examples of the malignant tumor include brain tumor, tongue cancer, laryngeal cancer, pharyngeal cancer, lung cancer, breast cancer, esophageal cancer, thyroid cancer, stomach cancer, pancreatic cancer, biliary tract cancer, duodenal cancer, colon cancer, liver cancer, renal cancer, uterine cancer, ovarian cancer, testicular cancer, prostate cancer, bladder cancer, osteosarcoma, chondrosarcoma, mesothelioma, skin cancer, leukemia, neuroblastoma, myeloma, and lymphoma.

The MRI contrast agent of the present invention can be administered either orally or parenterally. In the case of oral administration, the MRI contrast agent of the present invention can be administered in the form of, for example, a tablet, a capsule, granules, powder, or syrup. In the case of parenteral administration, the MRI contrast agent of the present invention can be administered in the form of, for example, an injection, a suppository, an eye drop, an inhalant, a transnasal agent, an ointment, or a cream. In the case of the injection, it is administered by, for example, intravenous administration, intramuscular administration, or intraperitoneal administration. These preparations can be produced by conventionally well-known methods using pharmaceutically acceptable additives such as an excipient, a lubricant, a binder, a disintegrator, a stabilizer, and a diluent.

Hereinafter, the present invention is described in further detail using examples. However, the range of the present invention is not limited by the following examples.
In the descriptions in the present specification, the following abbreviations are used:

THF: Tetrahydrofuran
BPE: (2-Bromo-2-methyl)propionyloxypropyltriethoxysilane
BPP: 1-(2-Bromo-2-methyl)propionyloxy-2-propene
PEGMA: Polyethylene glycol methacrylate
PEMA: (4-Hydroxyphenyl)ethyl methacrylate
dN-biby: Cu(I)Cl, Dinonyl bipyridine
EBIB: Ethyl 2-bromoisobutyrate
NMR: Nuclear Magnetic Resonance
GPC: Gel Filtration Chromatography
TGA: Thermogravimetric Analysis
TEM: Transmission Electron Microscope
MRI: Magnetic Resonance Imaging

[Examples]

<Production Example of Composite Particles (with microparticles being iron oxide nanoparticles)>

(1) Synthesis of a coupling agent containing a polymerization initiating group, (2-bromo-2-methyl)propionyloxypropyltriethoxysilane (BPE)

[Chemical Formula 2]

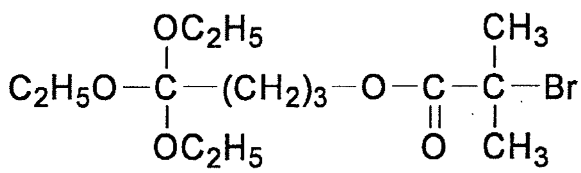

BPE

Synthesis of BPE was performed by a two-step reaction. As a first step, a mixed solution containing allyl alcohol (170 g), triethylamine (237 g), and dichloromethane (2 L) was cooled in ice and then 2-bromoisobutyryl bromide (450 g) was dripped therein. After that, the reaction solution was stirred at 0°C for three hours and was further stirred at room temperature for ten hours. The reaction solution was concentrated, THF was added thereto, and thereby salts were

allowed to precipitate and were then filtrated. After the filtrate was concentrated, the resultant was then diluted with chloroform (1 L). This was then washed with a 1N hydrochloric acid aqueous solution (2 × 1 L), a saturated sodium bicarbonate aqueous solution (2 × 1 L), and pure water (2 × 1 L) in this sequence. The organic layer was dried and was then concentrated. Thereafter, this was purified by vacuum distillation. Thus, 1-(2-bromo-2-methyl)propionyloxy-2-propene (BPP) was obtained at a yield of 90%.

As a second step, BPP (100 g), triethoxysilane (170 g), and a Karstedt catalyst (600 μL) were sequentially placed in a flask. The mixture thereof was stirred in an argon atmosphere at room temperature for 12 hours. After the reaction, it was purified by vacuum distillation and thus BPE was synthesized at a yield of 70%.

(2) Synthesis of Oleic Acid-Coated Iron Oxide Nanoparticles

In accordance with the report of Hyeon et al. (Nature Materials, Vol.3, p891-895, 2004), oleic acid-coated iron oxide nanoparticles were synthesized.

First, an iron oleate complex was synthesized. Ferric chloride ($FeCl_3 6H_2O$, 10.8 g) and sodium oleate (36.5 g) were dissolved in a mixed solvent of ethanol (80 mL), water (60 mL), and hexane (140 mL). The solution thus obtained was stirred at 70°C for four hours. After completion of the reaction, an upper organic layer was recovered and the organic layer was then washed with pure water three times. Thereafter, the organic layer was concentrated under reduced pressure to remove the solvent using a rotary evaporator. The residue thus obtained was vacuum-dried at 70°C overnight and thus an iron oleate complex was obtained. This iron oleate complex (36 g) and oleic acid (5.7 g) were dissolved in trioctylamine (200 g). The solution thus obtained was heated to 370°C at a heating rate of 5°C/min. The solution was further heated under reflux to maintain a temperature of 370°C for 30 minutes. Thereafter, the reaction solution was cooled to room temperature and after being diluted with THF, oleic acid-coated iron oxide nanoparticles were recovered by centrifugation (12,000 rpm). The projected area equivalent circular diameter (average particle size) of the iron oxide nanoparticles was measured by transmission electron microscopy and as a result, was found to be 7, 10, 15 and 20 nm. It was confirmed by the X-ray diffraction method that this iron oxide was magnetite ($Fe_3O_4$).

(3) Introduction of Initiating Group onto Surfaces of Oleic Acid-Coated Iron Oxide Nanoparticles

The iron oxide nanoparticles obtained in (2) were dispersed in THF and thereby a dispersion (1 wt%) was prepared. Aqueous ammonia (1 wt%) was added to the dispersion, which was then stirred for a while. Thereafter, the BPE (2 wt%) obtained in (1) was added thereto, which was then stirred at room temperature for three days. While being stirred, this solution was subjected to ultrasonic irradiation at regular intervals. Thereafter, the particles were recovered by centrifugation (12,000 rpm) and were then subjected to redispersion with THF and centrifugation repeatedly. Thus, iron oxide nanoparticles with initiating groups were obtained.

(4) Surface-Initiated Living Radical Polymerization Using Iron Oxide Nanoparticles

[Chemical Formula 3]

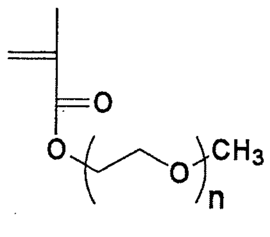

PEGMA

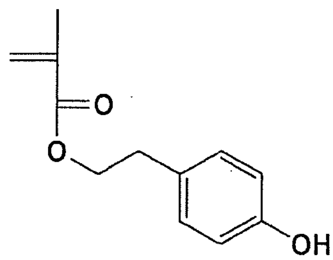

PEMA

The iron oxide nanoparticles with initiating groups obtained in (3), polyethylene glycol methacrylate (PEGMA), (4-hydroxyphenyl)ethyl methacrylate (PEMA), Cu(I)Cl, dinonyl bipyridine (dN-biby), and ethyl 2-bromoisobutyrate (EBIB) were placed in a Pyrex glass tube. This was degassed by the freezing and thawing method and was then sealed under vacuum. Thereafter, it was polymerized at 70°C for a predetermined period of time and thereby composite particles with polymer graft chains bound to the surfaces of the iron oxide nanoparticles were obtained. The polymerization conditions

are indicated in Table 1.

The composite particles (with the microparticles being the iron oxide nanoparticles) were purified by a repetition of centrifugation and redispersion into acetone and pure water.

The composite particles were dispersed in pure water, which was then stored at room temperature. The composite particles were treated with hydrogen fluoride and thereby the polymer graft chains were cut out from the iron oxide nanoparticles. Then the molecular weight and molecular weight distribution of the polymer graft chains were determined by gel filtration chromatography (GPC).

From the amount of grafts determined by thermogravimetric Analysis (TGA), the graft density was calculated. It was confirmed that all the samples had a high graft density of at least 0.15 to 0.43 chain/$nm^2$. Furthermore, the polymerization conditions used herein and polymerization results are indicated together in Table 1, which is intended to be used as a sample list. FIG. 1 shows a transmission electron microscope (TEM) photograph of composite particles (run 6).

[Table 1]

Table 1. Polymerization Conditions and Polymerization Results

| run | Polymerization Conditions for Polymer Graft Chains | | | | | | | Polymer Graft Chain Properties | | | |
|---|---|---|---|---|---|---|---|---|---|---|---|
| | EBIB : (PEGMA+PEMA) : Cu(I)Cl : dN-bipy | Monomer Amount (wt% | Iron Oxide Microparticle Size (nm) | Iron Oxide Microparticle Concentration (wt%) | Polymerization Solvent | Total Amount of Polymerization Solution (g) | Polymerization Time (h) | Number Average Molecular Weight (Mn) | Molecular Weight Distribution (Mw/Mn) | Number Average Degree of Polymerization | Graft Density (chain/ $nm^2$) |
| | Examples | | | | | | | | | | |
| 1 | 1 : 15000 : 150 : 300 | 50 | 7 | 0.25 | THF | 20 | 65.5 | 160,000 | 1.43 | 337 | 0.29 |
| 2 | 1 : 2000 : 10 : 20 | 50 | 7 | 0.25 | THF | 20 | 27 | 66,000 | 1.1 | 139 | 0.29 |
| 3 | 1 : 2000 : 10 : 20 | 50 | 10 | 0.5 | THF | 20 | 24 | 65,000 | 1.1 | 137 | 0.32 |
| 4 | 1 : 10000 : 100 : 200 | 50 | 10 | 0.5 | THF | 20 | 20 | 101,000 | 1.15 | 213 | 0.38 |
| 5 | 1 : 10000 : 100 : 200 | 50 | 10 | 0.5 | THF | 20 | 72 | 154,000 | 1.2 | 324 | 0.41 |
| 6 | 1 : 10000 : 100 : 200 | 50 | 20 | 0.5 | THF | 20 | 20 | 109,000 | 1.12 | 229 | 0.4 |
| 7 | 1 : 10000 : 100 : 200 | 40 | 20 | 0.5 | THF | 20 | 19.5 | 100,000 | 1.22 | 211 | 0.43 |
| 11 | 1 : 2000 : 10 : 20 | 50 | 15 | 0.5 | THF | 20 | 5.5 | 36000 | 1.30 | 76 | 0.15 |
| 12 | 1 : 2000 : 10 : 20 | 50 | 15 | 3 | THF | 50 | 9 | 57000 | 1.40 | 120 | 0.18 |
| | Comparative Examples | | | | | | | | | | |
| 8 | 1 : 2000 : 10 : 20 | 50 | 10 | 1 | THF | 10 | 16 | 29,000 | 1.13 | 61 | 0.11 |
| 9 | 1 : 100 : 0.5 :1 | 50 | 20 | 2 | THF | 10 | 4 | 11,000 | 1.22 | 23 | 0.06 |
| 10 | 1 : 100 : 0.5 : 1 | 50 | 20 | 0.5 | THF | 10 | 4 | 12,000 | 1.18 | 25 | 0.16 |

(continued)

| | | | | | | | | | | | |
|---|---|---|---|---|---|---|---|---|---|---|---|
| Comparative Examples | | | | | | | | | | | |
| 13 | 1 : 100 : 0.5 : 1 | 50 | 20 | 2 | THF | 10 | 4 | 26000 | 1.21 | 55 | 0.14 |

EBIB: Ethyl 2-bromoisobutyrate
PEGMA: Polyethylene glycol methacrylate
PEMA: (4-Hydroxyphenyl)ethyl methacrylate
dN-biby: Dinonyl bipyridine
THF: Tetrahydrofuran

<Measurement of Relaxation Level of Composite Particles>

With respect to the composite particles obtained in Example run 2 (7 nm), run 3 (10 nm), and run 6 (20 nm) that are different in particle size of the iron oxide microparticles from each other, the relaxation time was measured using NMR and MRI as follows.

Using NMR (EX-400, JEOL Ltd.), $T_2$ were measured by a CPMG (Carr-Purcell-Meiboom-Gill) method, with the measuring object being a proton signal of $H_2O$ in the presence of composite particles equivalent to 1 mM Fe (99% $D_2O$/24°C). $R_2$ determined from the result were 118 [Fe $mM^{-1} \cdot s^{-1}$] (run 2), 151 [Fe $mM^{-1} \cdot s^{-1}$] (run 3), and 326 [Fe $mM^{-1} \cdot s^{-1}$] (run 6). Accordingly, the composite particles obtained above were found to exhibit a $T_2$-shortening effect required as an MRI contrast agent. Furthermore, composite particles (run 6) were able to be obtained that indicated a $R_2$ of at least 200 [Fe $mM^{-1} \cdot s^{-1}$] and exhibited a high $T_2$-shortening effect even at a lower concentration. In the NMR used in this measurement, the magnetic field strength was 9.4 T, but $T_2$ relaxation is hardly affected by the difference in magnetic field strength (Questions & Answers in MAGNETIC RESONANCE IMAGING, Second Edition, published by Medical Science International).

Next, a phantom experiment was carried out using MRI (Unity INOVA 4.7T, Varian Technologies Japan Ltd.). The composite particles (run 6) were diluted with distilled water to a Fe concentration of 0.001 to 2.0 mM. These were placed as shown in the right-hand drawing of FIG. 2 and $T_2$ thereof were then measured by spin echo (measurement conditions: TR/TE = 4000/0.08 ms). The comparison subject used herein was an existing gadolinium-based contrast agent, Magnevist (MV, 1 mM Gd, Bayer Yakuhin, Ltd.) and the negative control used herein was distilled water. The numbers indicated in the circles shown in the right-hand drawing of FIG. 2 are concentrations (mM) of the composite particles, and MV indicates the existing gadolinium-based contrast agent used as the comparison subject. The left-hand drawing of FIG. 2 shows an MRI phantom image for measuring the relaxation level of the composite particles (run 6). As a result, with reference to the $T_2$ measurement image, the composite particles become dark in a concentration-dependent manner and it was confirmed that a sufficient MRI contrast image was able to be obtained even at lower concentrations as compared to the existing product. Furthermore, the same results were obtained also when other composite particles were used. The magnetic field strength was 4.7 T.

<Evaluation of Dispersion Stability of Composite Particles>

In order to evaluate the dispersion stability of the composite particles obtained in runs 1 to 13, a long-term storage test was carried out. Using PBS (phosphate buffered saline), the respective composite particles (Example runs 3, 4, and 5 as well as Comparative Example run 8; 5 mL each) with the iron oxide microparticles having a particle size of 10 nm were adjusted to have a particle concentration of 0.038 wt% and were then stored at room temperature for six months. Before the start of storage, no aggregation of any composite particles was found. As a result of dynamic light scattering particle size distribution measurement using LB-550 (manufactured by HORIBA, Ltd.), the average particle sizes (hydrodynamic diameter; the median size was used in the present measurement) were smaller than 40 nm, and all of them exhibited excellent dispersion stability. When these composite particles were stored at room temperature for 6 months, precipitation occurred only in Comparative Example run 8 (FIG. 3). Similarly, the measurement of particle size distribution of the composite particles was carried out and as a result, Comparative Example run 8 was 296 nm, run 3 was 120 nm, run 4 was 75 nm, and run 5 was 24 nm. Thus, the longer the graft chain length was, the more the dispersibility obtained before the start of storage was maintained. From these results, it was confirmed that composite particles with higher graft chain molecular weights were excellent in dispersion stability and were suitable for a long period of storage. This is a surprising result indicating that composite particles with polymer graft chains bound thereto by living radical polymerization exhibit a characteristic that the dispersion stability is maintained for a long period of time even when they are simply mixed in an aqueous solvent.

<Phagocytosis of Composite Particles by Mouse Intraperitoneal Macrophages>

In order to evaluate the tendency of being taken into the reticuloendothelial system of the composite particles, the in vitro macrophage test was carried out. A mouse (8 weeks old, ICR male mouse) subjected to intraperitoneal administration of 2 mL of 3% thioglycolate (manufactured by SIGMA CORPORATION) was sacrificed after three days and macrophages were recovered from the abdominal cavity using Hanks' solution (manufactured by Gibco Corporation). The macrophages thus recovered were adjusted to have a $1 \times 10^6$ cells/mL after being washed with Hanks' solution and a culture medium (DMEM, manufactured by Gibco Corporation) and then cultivated under the conditions of 37°C and 5 % $CO_2$. After three hours, the culture medium was removed and composite particles (Example runs 1 to 7, 11, and 12 as well as Comparative Example runs 8 to 10 and 13) adjusted to have 30 $\mu$g Fe/mL using the culture medium were added thereto. After 24 hours of cultivation, the culture medium was removed therefrom, which was then washed with PBS three times. Thereafter, the macrophages that phagocytized the composite particles were stained with Perl's Prussian Blue stain and the absorbance (590 nm) was measured (FIG. 4). The negative control used herein was PBS and the positive control used herein was an existing iron oxide contrast agent, Resovist (Bayer Ltd.; 30 μg Fe/mL). As a result, when the composite particles of Example were used, there was no difference from the negative control and therefore it was confirmed that they were not phagocytized by macrophages. Generally, it is considered that when the composite particles have higher graft chain molecular weights, the density at the graft chain end decreases as compared to the vicinity where the microparticles were bound, and they tend to be recognized by the reticuloendothelial system and thus tend to be phagocytized. Therefore, it was considered that the composite particles with lower graft chain molecular weights tended not to be phagocytized by macrophages. Contrary to expectations, however, it was confirmed that as compared to the composite particles (Comparative Example) with lower graft chain molecular weights, the composite particles (the present invention) with higher graft chain molecular weights tended not to be phagocytized by macrophages.

<Phagocytosis of Composite Particles by Mouse Liver Macrophages>

In order to evaluate the tendency of being taken into the liver of the composite particles, the histopathological evaluation of the liver was carried out. Each solution of the composite particles (Example run 5 and Comparative Example run 8; 300 μL each) with a concentration adjusted to 1.9 wt% using a normal saline solution was administered intravenously through the tail of a mouse (6 weeks old, ICR male mouse). They were sacrificed three days after administration and the extirpated livers were formalin-fixed. Thereafter, they were paraffin-embedded, sectioned, and then were HE-stained. As a result of the histopathological evaluation of the liver, in the case of Comparative Example run 8 (the particle size of the iron oxide microparticles: 10 nm, Mn: 29,000), the liver macrophages (Kupffer cells) shown in the circles were stained a brownish color, and thereby it was indicated that the iron oxide nanoparticles (composite particles) were phagocytized (FIG. 5B). In the case of run 5 (the particle size of the iron oxide microparticles: 10 nm, Mn: 154,000), only normal Kupffer cells shown in the circles were found and no phagocytosis was observed (FIG. 5A). From this result, it was confirmed that the composite particles of Example were not phagocytized by the Kupffer cells in the liver reticuloendothelial system and were not accumulated in the liver three days after administration.

<Pharmacokinetics Evaluation of RI-Labeled Composite Particles>

(1)Labeling of Composite Particles with Radioactive Isotope (RI)

Dispersions (the concentration of the composite particles: 1 wt%, the solvent = pure water, 100 μL) of the composite particles of Example run 6 (the particle size of iron oxide microparticles: 20 nm, Mn: 109,000) and Comparative Example run 9 (the particle size of iron oxide microparticles: 20 nm, Mn: 11,000) each were placed in a microcentrifuge tube, and further $Na^{125}I$ (5 pL, Perkinelmer NEZ033) and then a Chloramine T solution (the concentration: 0.2 mg/m1, the solvent = 0.5M phosphate buffered aqueous solution (pH 7.5, containing 0.5M NaCl, 100 μL) were added thereto. Each solution thus obtained was stirred with a vortex mixer for two minutes. Further, a sodium metabisulfite solution (the concentration: 4 mg/mL, the solvent = pure water, 100 μL) was added thereto, which was then stirred with the vortex mixer for two minutes. The mixture thus obtained was separated and purified (the eluate: normal saline solution) with a PD-10 column (GE Healthcare Japan Corporation) and thereby a distillation containing microparticles ($^{125}I$-labeled composite particles) were recovered as a $^{125}I$-labeled composite particle dispersion.

(2) Pharmacokinetics Evaluation of RI-Labeled Composite Particles

The $^{125}I$-labeled composite particle dispersions (runs 6 and 9, 100 μL each) produced in (1) each were administered intravenously through the tail of a tumor-bearing mouse (8 weeks old, BALB/c female mouse) with Colon-26 tumor cells transplanted thereinto. After the lapse of a predetermined period of time, blood was collected from a mouse eye. The radioactivity thereof was then measured with a gamma counter (ARC-301B, ALOKA CO., LTD.) and thereby the percentage of the composite particles remaining in the blood was determined (FIG. 6). Furthermore, the tumor-bearing mice were sacrificed 24 hours after administration. The radioactivity of each organ extirpated therefrom was measured and thereby the biodistribution of the composite particles was evaluated (FIG. 7A; the rate of accumulation in tissue, FIG. 7B: the rate of accumulation in tissue per weight). As a result of the percentage of the composite particles remaining in the blood, in Comparative Example run 9 (the particle size of the iron oxide microparticles: 20 nm, Mn: 11,000), the percentage decreased from immediately after administration, while in Example run 6 (the particle size of the iron oxide microparticles: 20 nm, Mn: 109,000), a high percentage (approximately 40%) of the composite particles remaining in the blood was maintained up to 24 hours after administration, which indicated a high retention in blood. In the biodistribution, with respect to the rate of accumulation in tissue measured 24 hours after administration, an accumulation of 8 to 18% in liver and an accumulation of 1 to 2% in spleen were observed in both trial products. Since the

rate of accumulation in the liver reticuloendothelial system measured one hour after administration of a conventional iron oxide-based contrast agent was approximately 80% (Am J Roentgenol, 152, 167-173, 1989), it was confirmed that the rates of accumulation of the composite particles of Example in the liver and the spleen were very low. Furthermore, in the rate of accumulation in tissue per weight measured 24 hours after administration of Example run 6, a high rate of accumulation in the tumor was proved. From these results, it was confirmed that in the composite particles with higher graft chain molecular weights, the retention in blood was prolonged and accumulation in the tumor increased due to the EPR effect, while the rates of accumulation in the liver and the spleen were low.

<Evaluation of Imaging of Composite Particles by MRI>

Using the composite particles of Example run 6 (the particle size of iron oxide microparticles: 20 nm, Mn: 109,000), an MRI test was carried out. A tumor-bearing mouse (8 weeks old, BALB/c female mouse) under anesthesia was set in an MRI system (Unity INOVA 4.7T, Varian Technologies Japan Ltd.). After the tumor of the left femoral region was positioned and imaged (before administration) (see FIGS. 8A and 8B), a dispersion (100 pM Fe/kg, 100 μL) of composite particles of run 6 prepared with a normal saline solution was intravenously administered through the tail and the same tumor was imaged after 24 hours (see FIG. 8C). The imaging was carried out under the conditions including: the transverse plane, 1 mm thick, TR = 300 ms, TE = 10 ms, and a flip angle of 20°. As a result, in the $T_2$-weighted image that was imaged by the gradient echo (GE) measurement, the interior of the tumor, which was imaged as a white image before administration of the composite particles, was shadowed overall and particularly in a part thereof a perfect shadow effect was observed in the image 24 hours after administration (see FIG. 8C). This result showed that the composite particles accumulated in the tumor and thereby exhibited the $T_2$-shortening effect to reduce the signal strength. Accordingly it was confirmed that the composite particles practically function as an MRI contrast agent that enhances contrast.

Industrial Applicability

The MRI contrast agent of the present invention is also applicable for use as a nano-drug carrier, or an imaging reagent.

## Claims

1. An MRI contrast agent comprising composite particles,
wherein the composite particles are those with polymer graft chains bound to surfaces of microparticles at a very high density sufficient to allow steric repulsion to occur between the graft chains, the microparticles are inorganic microparticles that exhibit superparamagnetism, the polymer graft chains have a number average molecular weight (Mn) of 30,000 to 320,000, a graft density of 0.15 to 0.45 chain/$nm^2$, and a molecular weight distribution of 1 to 1.5.

2. The MRI contrast agent according to claim 1, wherein the polymer graft chains are obtained by living radical polymerization of at least one selected from the group consisting of an acrylic acid derivative, a methacrylic acid derivative, an acrylamide derivative, a methacrylamide derivative, and a styrene derivative, with polymerization initiating groups located on the surfaces of the microparticles being used as base points.

3. The MRI contrast agent according to claim 1 or 2, wherein the MRI contrast agent is for use for tumor diagnosis.

4. Use of an MRI contrast agent according to claim 1 or 2 in MRI.

## Patentansprüche

1. Ein MRT-Kontrastmittel, umfassend Verbundpartikel,
wobei die Verbundpartikel solche sind, bei denen Polymerpfropfketten an Oberflächen von Mikropartikeln mit einer sehr hohen Dichte gebunden sind, die ausreicht, um eine sterische Abstoßung zwischen den Pfropfketten zu ermöglichen, die Mikropartikel anorganische Mikropartikel sind, die Superparamagnetismus zeigen, die Polymerpfropfketten ein Zahlenmittel des Molekulargewichts (Mn) von 30.000 bis 320.000, eine Pfropfdichte von 0,15 bis 0,45 Kette/$nm^2$ und eine Molekulargewichtsverteilung von 1 bis 1,5 aufweisen.

2. Das MRT-Kontrastmittel nach Anspruch 1, wobei die Polymerpfropfketten durch lebende radikalische Polymerisation

von mindestens einem, ausgewählt aus der Gruppe bestehend aus einem Acrylsäurederivat, einem Methacrylsäurederivat, einem Acrylamidderivat, einem Methacrylamidderivat und einem Styrolderivat, erhalten werden, wobei Polymerisation-initiierende Gruppen, die sich auf den Oberflächen der Mikropartikel befinden, als Basispunkte verwendet werden.

**3.** Das MRT-Kontrastmittel nach Anspruch 1 oder 2, wobei das MRT-Kontrastmittel zur Verwendung bei der Tumordiagnose bestimmt ist.

**4.** Verwendung eines MRT-Kontrastmittels nach Anspruch 1 oder 2 bei der MRT.

## Revendications

**1.** Agent de contraste d'IRM comprenant des particules composites,
où les particules composites sont celles avec des chaînes greffées polymères liées à des surfaces de microparticules à une densité très élevée suffisante pour permettre qu'une répulsion stérique ait lieu entre les chaînes greffées, les microparticules sont des microparticules inorganiques qui présentent un superparamagnétisme, les chaînes greffées polymères présentent une masse moléculaire moyenne en nombre (Mn) de 30 000 à 320 000, une densité de greffage de 0,15 à 0,45 chaine/nm$^2$, et une distribution de masse moléculaire de 1 à 1,5.

**2.** Agent de contraste d'IRM selon la revendication 1, où les chaînes greffées polymères sont obtenues par polymérisation radicalaire vivante d'au moins un choisi dans le groupe constitué d'un dérivé d'acide acrylique, d'un dérivé d'acide méthacrylique, d'un dérivé d'acrylamide, d'un dérivé de méthacrylamide, et d'un dérivé de styrène, avec des groupes d'initiation de polymérisation disposés sur les surfaces des microparticules utilisées comme points de base.

**3.** Agent de contraste d'IRM selon la revendication 1 ou 2, où l'agent de contraste d'IRM est destiné à une utilisation pour un diagnostic de tumeur.

**4.** Utilisation d'un agent de contraste d'IRM selon la revendication 1 ou 2 dans une IRM.

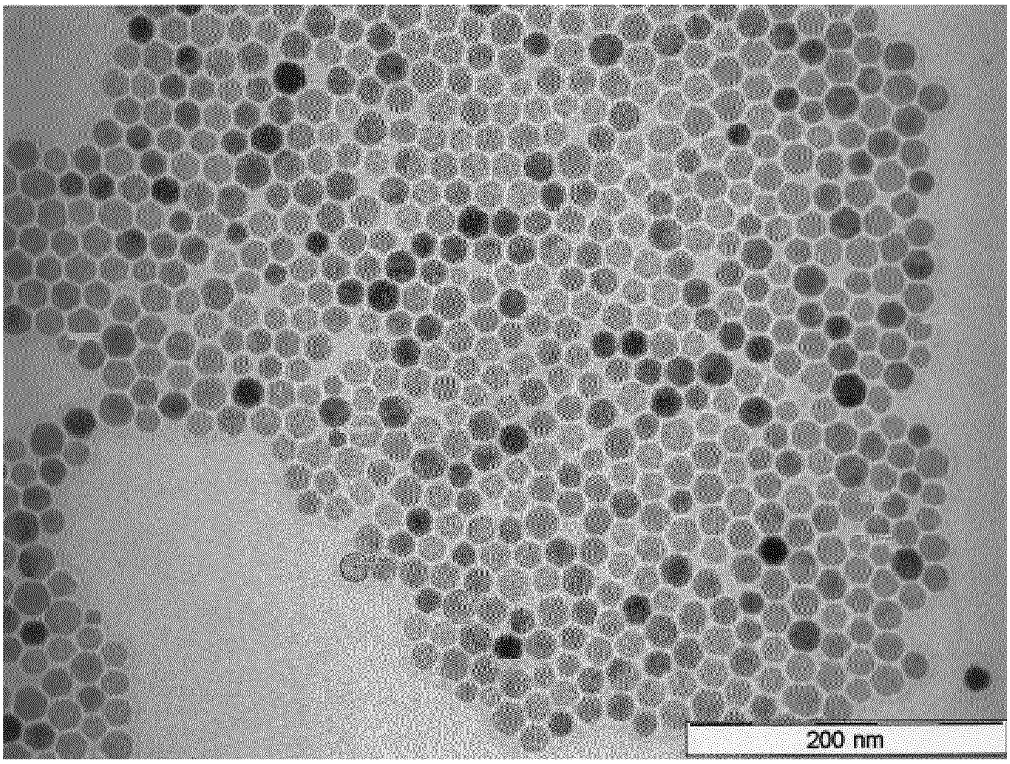


FIG. 1

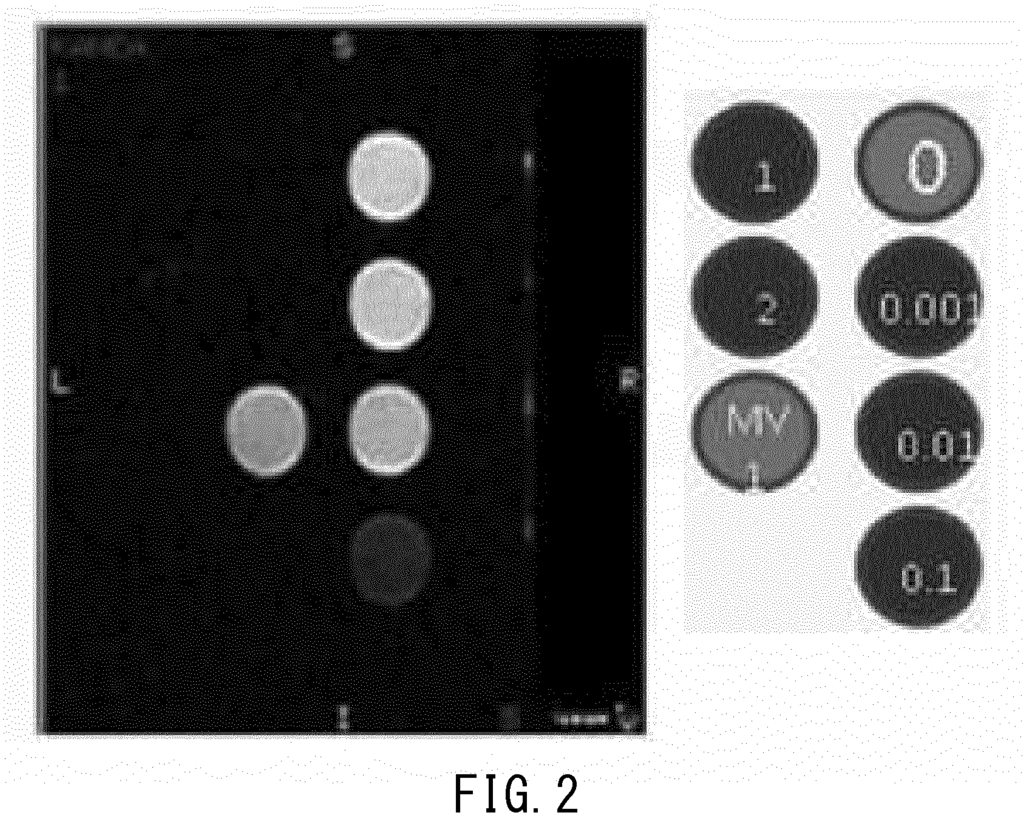


FIG. 2

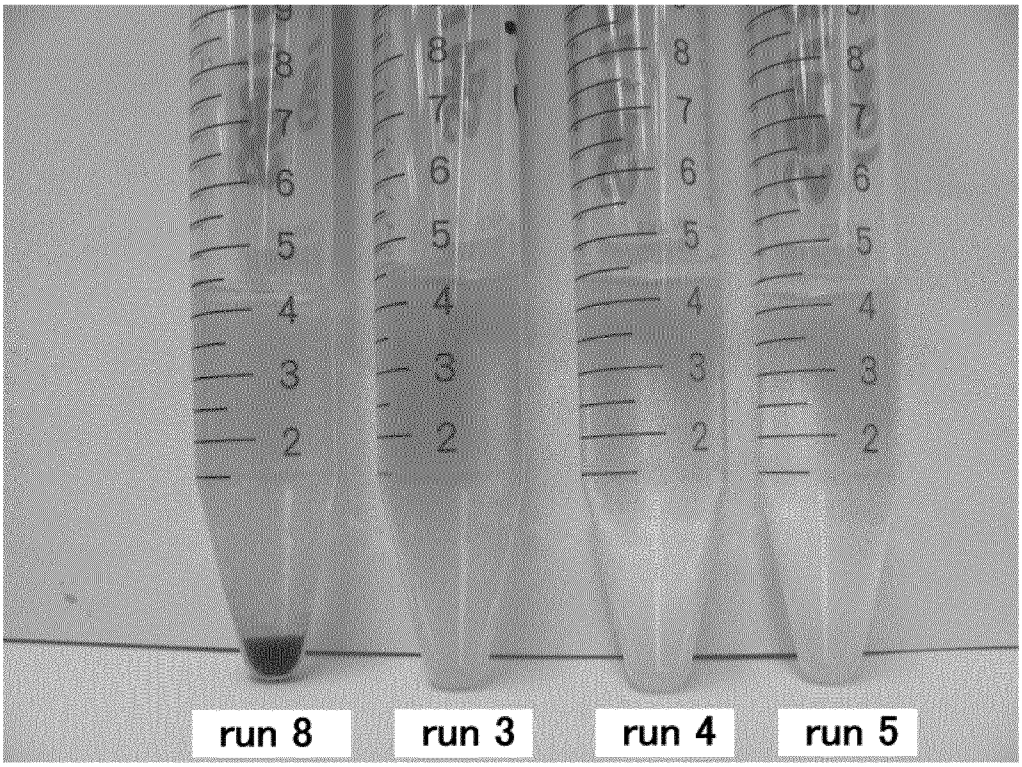
run 8
run 3
run 4
run 5

FIG. 3

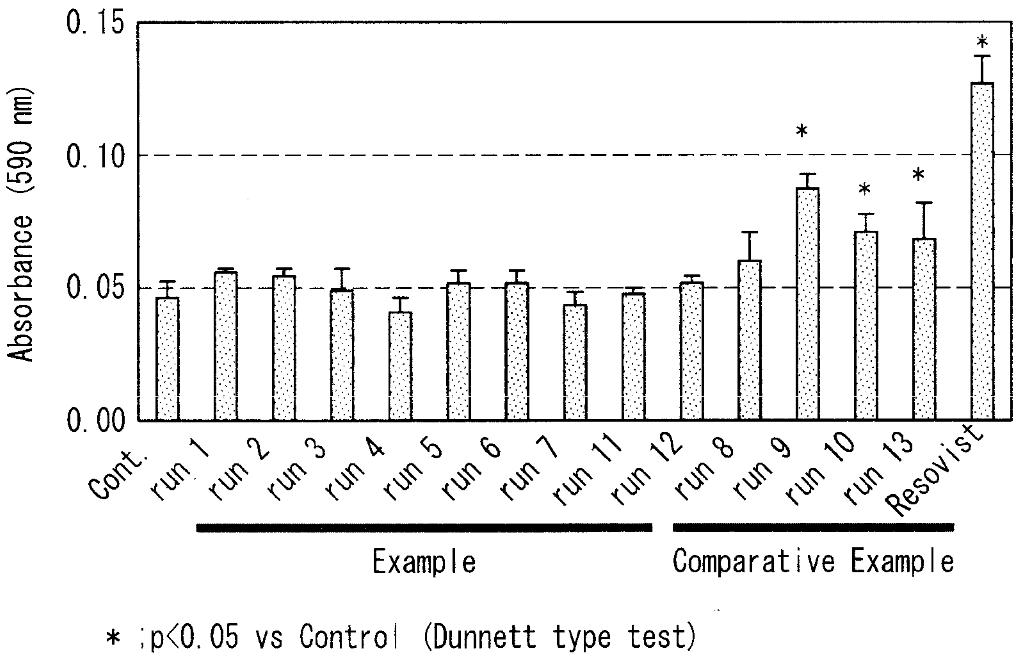
0.15
0.10
0.05
0.00
Absorbance (590 nm)
Cont.
run 1
run 2
run 3
run 4
run 5
run 6
run 7
run 11
run 12
run 8
run 9
run 10
run 13
Resovist
Example
Comparative Example
* ;p<0.05 vs Control (Dunnett type test)

FIG. 4

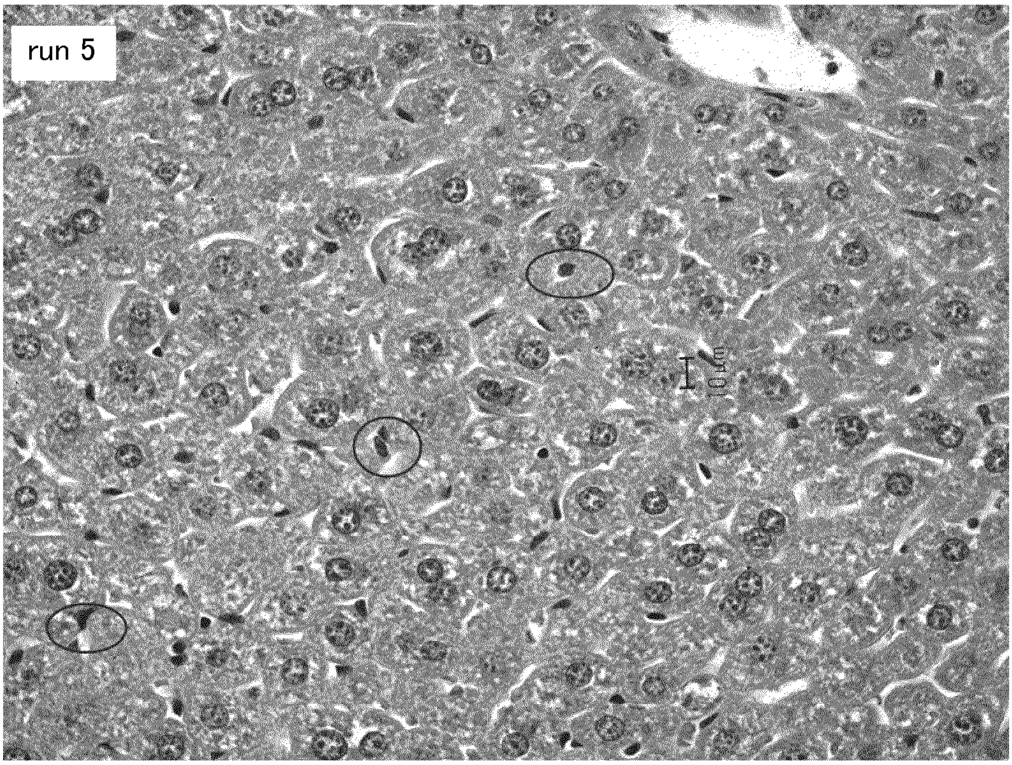


FIG. 5A

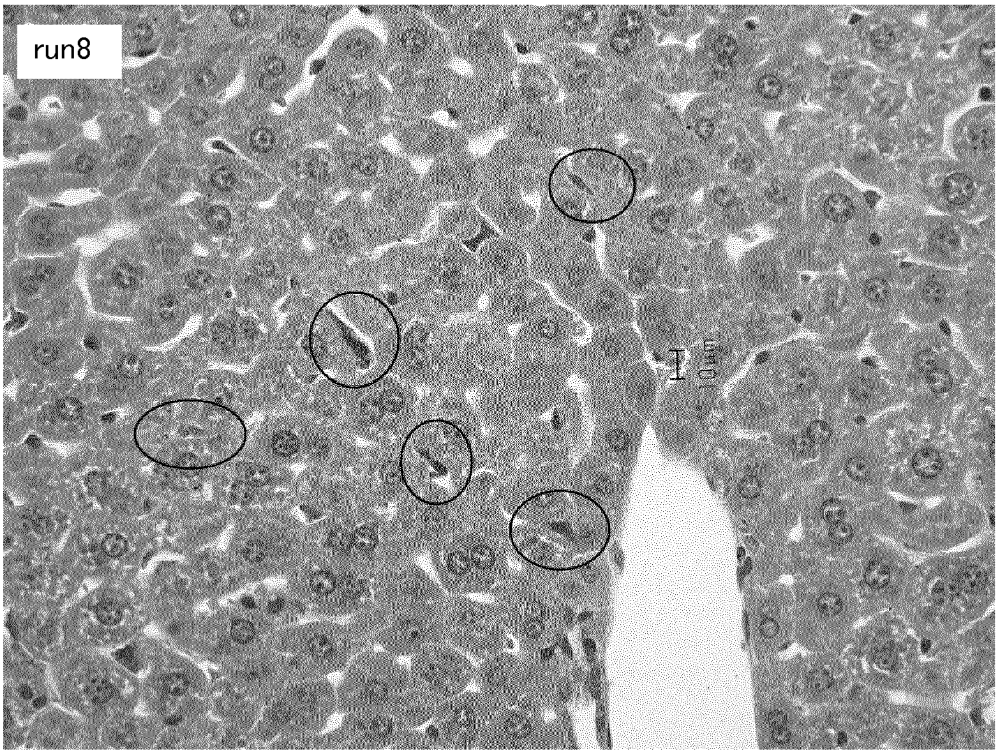


FIG. 5B

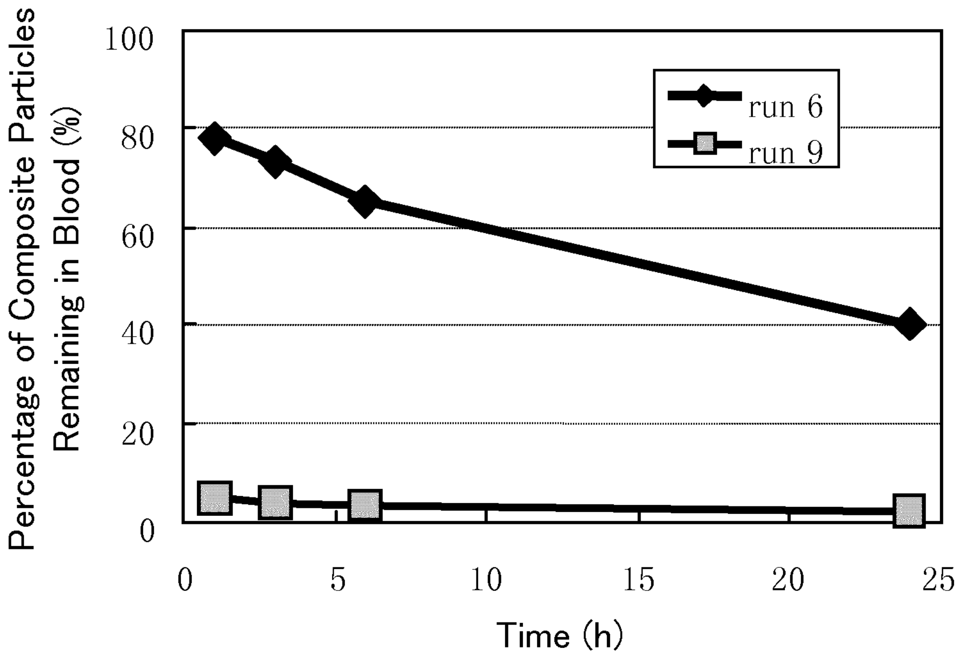
Percentage of Composite Particles
Remaining in Blood (%)
100
80
60
40
20
0
0
5
10
15
20
25
Time (h)
run 6
run 9

FIG. 6

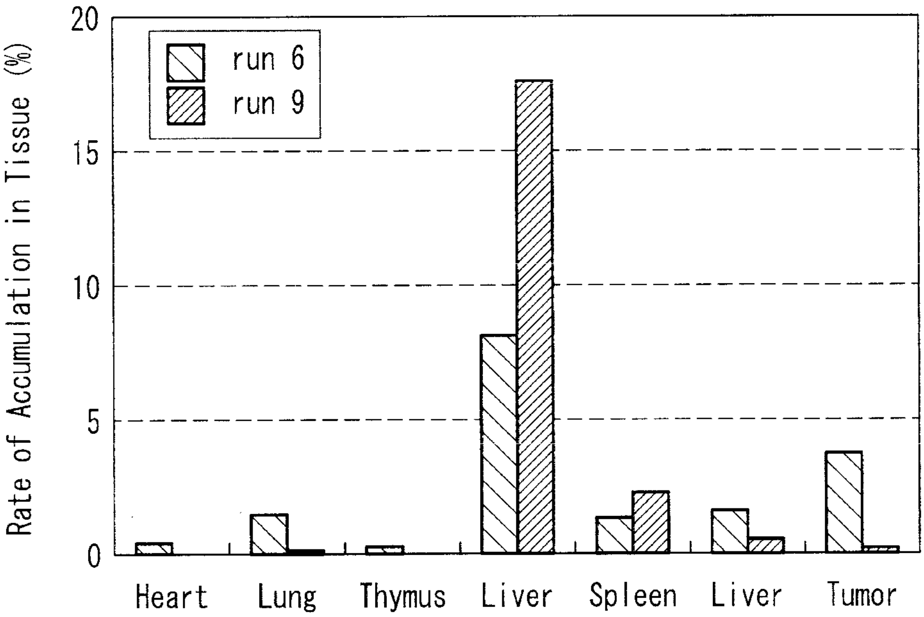
Rate of Accumulation in Tissue (%)
20
15
10
5
0
run 6
run 9
Heart
Lung
Thymus
Liver
Spleen
Liver
Tumor

FIG. 7A

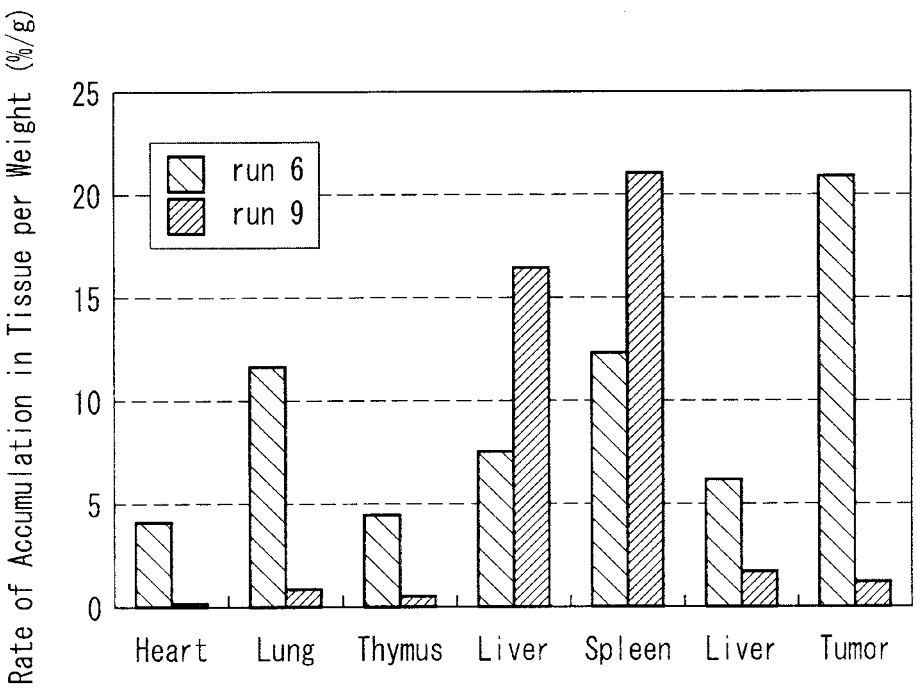
Rate of Accumulation in Tissue per Weight (%/g)
25
20
15
10
5
0
run 6
run 9
Heart
Lung
Thymus
Liver
Spleen
Liver
Tumor

FIG. 7B

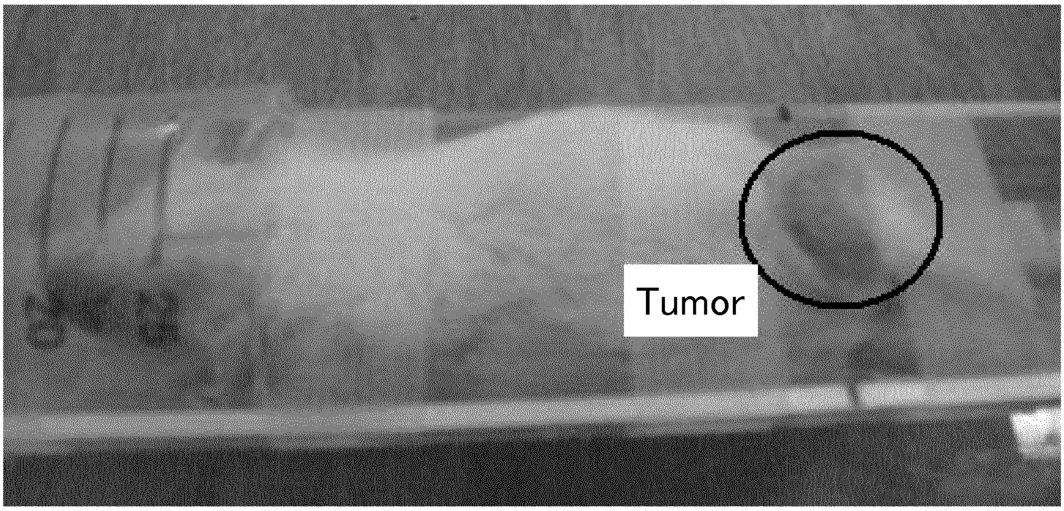


FIG. 8A

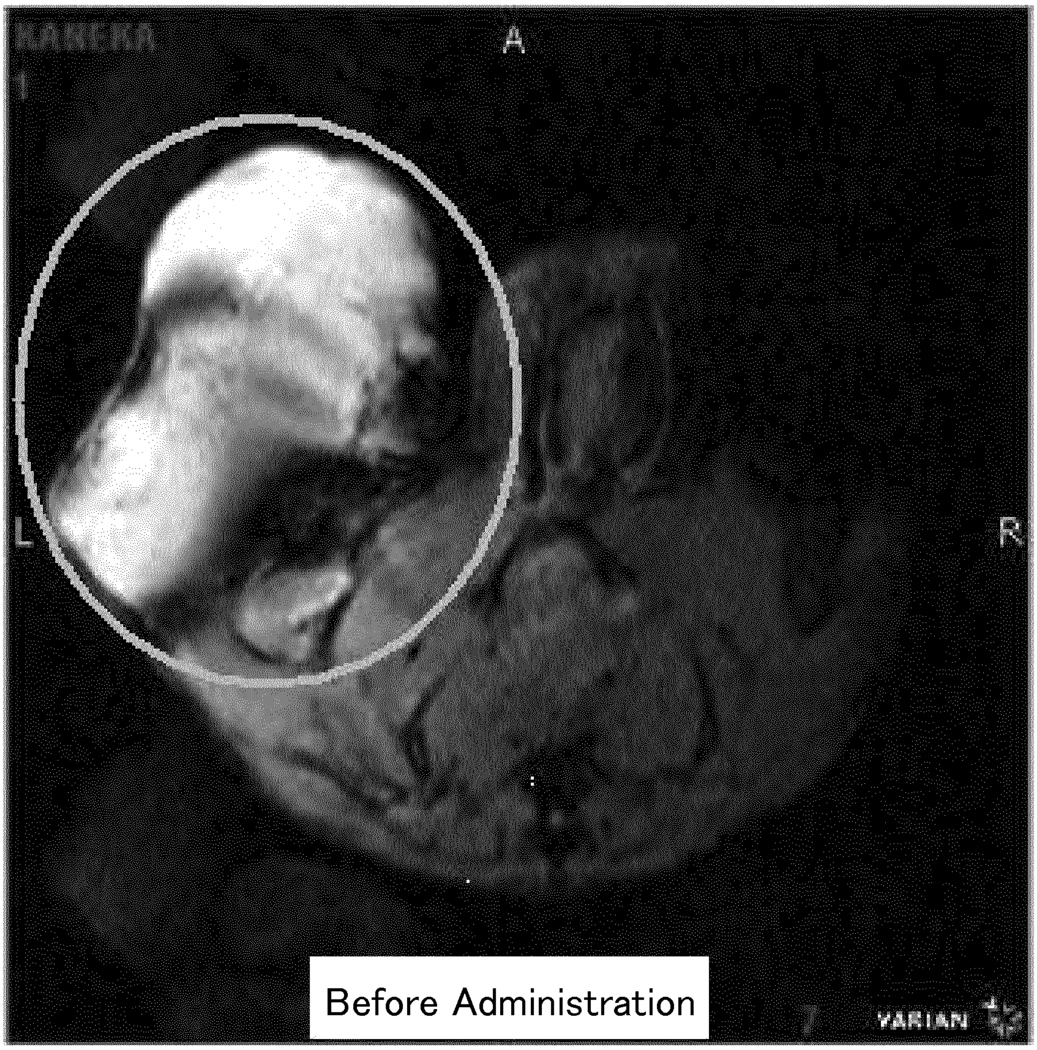


FIG. 8B

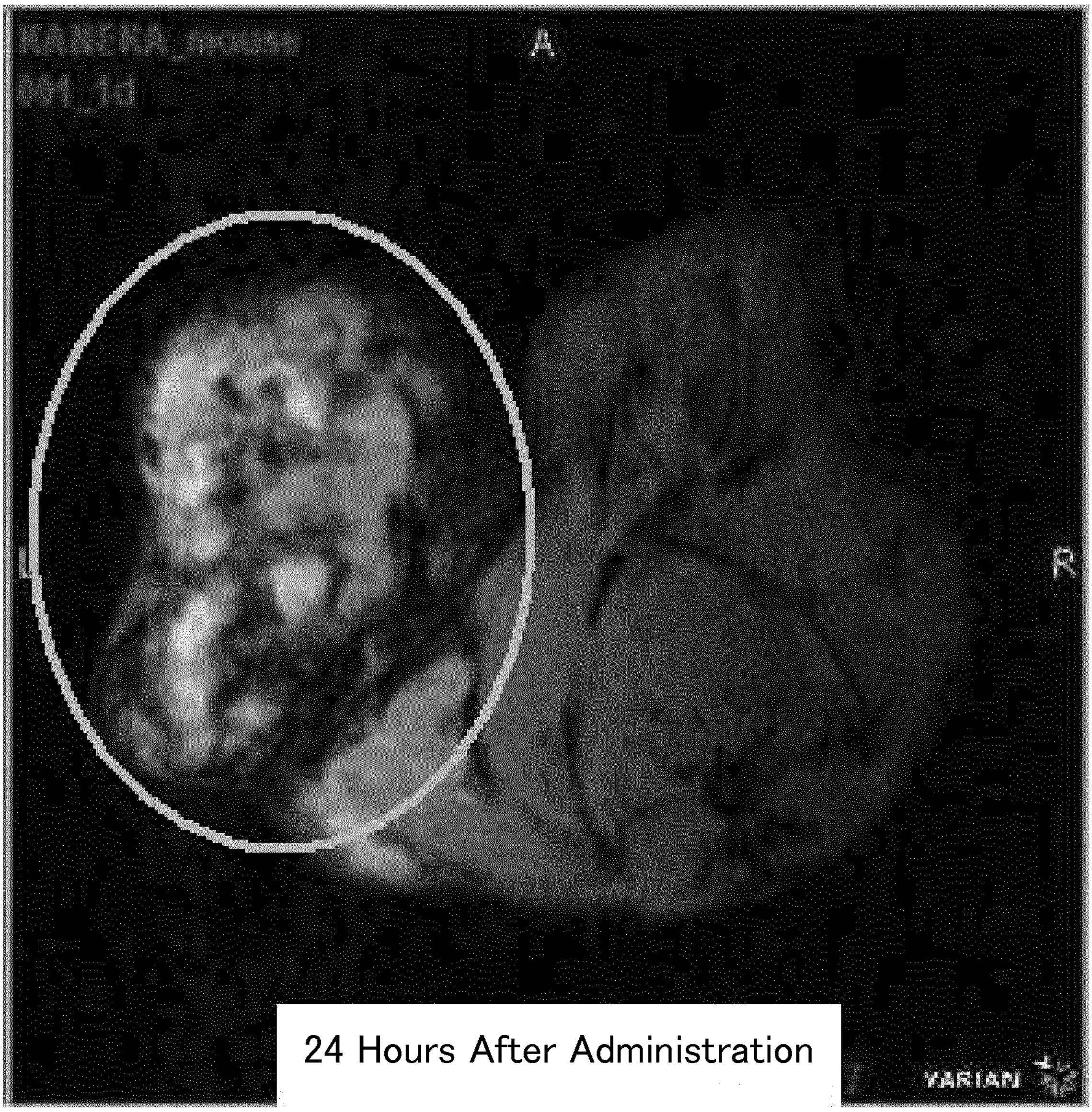
KANEKA_mouse
001_1d
A
L
R
24 Hours After Administration
VARIAN

FIG. 8C

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*



### Patent documents cited in the description


- WO 2006068653 A
- JP 2009519316 A


### Non-patent literature cited in the description


- *BIOMACROMOLECULES,* 14 September 2009, vol. 10 (9), 2662-2671
- *Macromolecules,* 2009, vol. 42 (4), 1219-1228
- *Advanced Drug Delivery Reviews,* 2006, vol. 58, 1471-1504
- **HYEON et al.** *Nature Materials,* 2004, vol. 3, 891-895
- *Am J Roentgenol,* 1989, vol. 152, 167-173